# EUROPEAN PATENT SPECIFICATION

(11) **EP 1 841 437 B1**
(45) Date of publication and mention of the grant of the patent: **29.04.2009**
(21) Application number: 06744666.6
(22) Date of filing: 12.01.2006
(51) Int. Cl.: A61K 31/7048, A61P 33/06

(54) **9A-CARBAMOYL AND THIOCARBAMOYL AZALIDES WITH ANTIMALARIAL ACTIVITY**
9A-CARBAMOYL- UND THIOCARBAMOYLAZALIDE MIT ANTIMALARIAWIRKUNG
AZALIDES 9A-CARBAMOYLE ET THIOCARBAMOYLE AVEC ACTIVITE ANTIPALUDIQUE

(30) Priority: 14.01.2005 US 644350 P
(43) Date of publication of application: 10.10.2007
(73) Proprietor: GlaxoSmithKline istrazivacki centar Zagreb d.o.o., 10000 Zagreb (HR)
(72) Inventor: ALIHODZIC, Sulejman, Prilaz baruna 29 (HR); MUTAK, Stjepan, Prilaz baruna 29 10000 Zagreb (AT); IVEZIC, Zrinka, Prilaz baruna 29 10000 Zagreb (HR)
(74) Representative: Crawley, Karen Anne
(86) International application number: PCT/IB2006/001189
(87) International publication number: WO 2006/087642

(56) References cited:
- EP-A- 0 657 464
- EP-A- 1 253 153
- WO-A-00/57866
- WO-A-00/66603
- WO-A-98/56802
- WO-A-02/068438
- WO-A-20/04043985
- WO-A-20/04101591
- SADIQ ET AL: "Effects of azithromycin on malariometric indices in The Gambia" LANCET, vol. 346, 1995, pages 881-882, XP009104211

## Description

### Field of the Invention

The present invention relates to the use of 9a-carbamoyl and thiocarbamoyl azalides and their pharmaceutically acceptable derivatives for the treatment and prevention of malaria.

### Technical Problem

The invention is directed to the use of antimalarial agents not heretofore used to treat malaria, to which the malarial parasites have not developed resistance.

### Background of the Invention

Malaria is a serious infection. 200 to 300 million people are infected with malaria and two to three million people die from malaria every year. The disease is caused by a parasite (a protozoa of the Plasmodia genus), which is transmitted by the female *Anopheles* mosquito. There are four parasites hat can effect humans, *Plasmodium falciparum, P. vivax, P. ovale,* and *P. malariae*. A distinction is drawn between *Malaria tropica* (caused by *Plasmodium falciparum*), *Malaria tertiana* (caused by *Plasmodium vivax* or *Plasmodium ovale*) and *Malaria quartana* (caused *by Plasmodium malariae). Malaria tropica* is the most severe form of the disease, and is characterized by severe constitutional symptoms, and sometimes causes death.

Malaria is characterized by attacks of chills, fever, and sweating, occurring at intervals which depend on the time required for development of a new generation of parasites in the body. After recovery from the acute attack, the disease has a tendency to become chronic, with occasional relapses. The disease is prevalent in tropical and subtropical areas of the world including the Amazon region of Brazil, East and Southern Africa and Southeast Asia. The emergence of a malaria parasite resistant to chloroquine, is a drug heavily used as a panacea of malaria, has become a serious problem, and therefore, there is an urgent need to develop an effective remedy. Also, attempt to develop a malaria vaccine have failed. This compounds the urgent need to find an alternative drug-based approach to treating malaria.

Drugs of diverse chemical classes, such as chloroquine, mefloquine, halofantrine, and artemisinin, atovaquone/proguanil (Malarone^{™} ), doxycycline, and primaquine have been developed for the treatment of malaria. However, while marginally successful against some strains of malaria, most strains of malaria appear to have developed resistance not only to individual drugs but also to multiple combinations of drugs. Drugs which worked initially become totally ineffective after a period of time. An initial period of remission is often followed by a period during which nothing seems to be effective against the disease. This is known as multiple drug resistance, and it remains an issue in antimalarial drug development efforts. A malarial parasite which initially responds to treatment by one or more drugs becomes resistant to treatment not only using the drugs previously used, but many other antimalarial drugs. This further underscores the urgent necessity to find new compounds which show good efficacy against malaria and minimal toxicity.

In the last years several reports indicated that macrolides have potential for prophylactic as well as therapeutic use against malaria. Midecamycinin was studied in 1989 in two infectious models using *Plasmodium berghei* and *Plasmodium yoelii nigeriensis* (mouse) and *Plasmodium cynomolgi* (rhesus monkey) [S. K. Puri and G. P. Duti, Chemotherap.35 (1989) 187]. In both mouse models, midecamycinin was active. The doses for *Plasmodium berghei* infection were significantly lower than for *Plasmodium yoelii nigeriensis*. In the monkey model, no efficacy was noted. In other investigation the animal model was challenged with azihromycin [S. K. Puri and N. Singh, Exp. Parasitol. 94 (2000) 8]. The dose regimen of 25-50 mg/kg reflects the same dose used for antibacterial treatment. Azithromycin worked in prophylactic and therapeutic dosing and in contrast to midecamycinin azithromycin was active also in the monkey model.

The efficacy of azithromycin in treating to malarial infections was studied in Gambia [S. T. Sadiq et al, Lancet 1995 30, 346,881]. Children undergoing therapy for trachoma (Azithromycin is highly effective against *C. trachomati )* were also examined for signs of malaria prophylaxis or therapeutic effects. A clear improvement of various indicators of malaria infection suggested a significant therapeutic benefit of azithromycin. The prophylactic efficacy of azithromycin was confirmed in Kenya [S. L. Anderson et al., Ann. Intern. Med. 123, 771]. A significant protection in adult volunteers was achieved with a better prophylaxis obtained through use of a daily dosing scheme of 250 mg versus a weekly regimen of 1000 mg. Also, in a double-blind, placebo-controlled trial with azithromycin in Irian Jaya in Indonesia [W. R. Taylor et al., Clin. Infect. Dis. 28 (1999) 522], the prophylactic efficacy in azithromycin treated non-immune patients was 71.6 % for *Plasmodium falciparum* and 98,9 % for *Plasmodium vivax* as compared to controls.
EP1253153 and WO98/56802 disclose processes for the preparation of 9-deoxo-9a-aza-9a-homoerythromycin derivatives, which are useful for treating infections of bacteria or protozoa. WO00/57866 discloses the use of azithromycin derivatives for treating ocular infections caused by, *inter alia* malaria.
WO00/66603, EP0657464, WO04/43985, WO02/68438 and WO04/101591 disclose 9a-carbamoyl and thiocarbamoyl azalides for use in treating bacterial infection.

### Summary of the Invention

It has been found that 9a-carbamoyl and thiocarbamoyl azalides have antimalarial activity. These compounds can therefore be used in the treatment and prevention of malaria.

The present invention is directed to the use of 9a-carbamoyl and thiocarbamoyl azalides represented by the general formula **(I),** 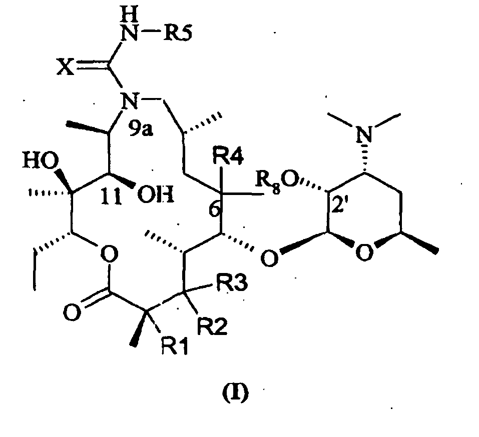 wherein:
- R¹: is hydrogen or together with R² is a double bond;
- R²: is a cladinose sugar of formula **(II),** hydrogen, hydroxyl or group of the formula **(III)** wherein **Y** is a monocyclic aromatic ring unsubstituted or substituted
with one or more groups which are selected from halogen, OH, OMe, NO₂, and NH₂; or 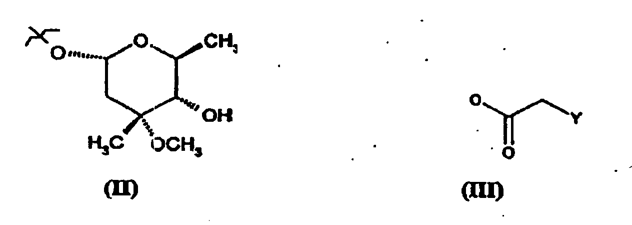
- R²: together with R³ is a ketone, or together with R¹ is a double bond;
- R³: is hydrogen or together with R² is a ketone, or together with R⁴ is an ether;
- R⁴: is hydroxyl or OMe, or together with R³ is an ether;
- R⁵: is C₁₋₄alkyl, C₂₋₄alkenyl, -(CH₂)ₘ-Ar, wherein Ar is a monocyclic or bicyclic aromatic ring having up to 10 carbon atoms containing 0-3 heteroatoms selected from N and
- O,: unsubstituted or substituted by one or more of halogen, C₁₋₆haloalkyl, C₁₋₆haloalkoxy, C₁₋₆alkyl, or C₁₋₆alkoxy, and m is 0-3; or
- R⁵: is a substituted aryl group of the formula **(IV),** 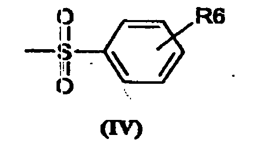 wherein R⁶ is H, C₁₋₄ alkyl or halogen,
or,
- R⁵: is a substituted aryl group of the formula (V), 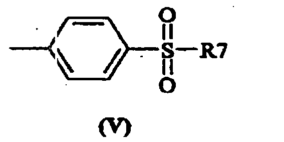 wherein R⁷ is chloro, amino phenylamino, 2-pyridylamino, 3,4-dimethylisoxazolyl-5-amino, or 5-methylisoxazolyl-3-amino;
- R⁸: is hydrogen or hydroxyl protecting group; and
- X: is oxygen or sulfur;
or a pharmaceutically acceptable salt, solvate or ester thereof, in the manufacture of a medicament for the therapeutic and/or prophylactic treatment of malaria.

### Detailed Description of the Invention

In some embodiments, the use comprises use of a compound of Formula (I), wherein
- R¹: is hydrogen;
- R²: is hydroxyl, or a cladinose sugar of formula (II); 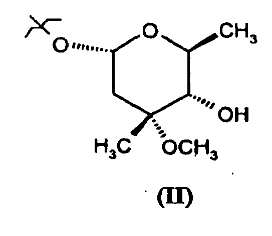
- R³: is hydrogen;
- R⁴: is hydroxyl,;
- R⁵: is isopropyl, benzyl, 3-chlorophenyl or 1-naphthyl; or
- R⁵: is a substituted aryl group of the formula (IV), 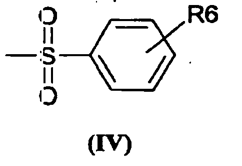 wherein R⁶ is 2-chloro,
or,
- R⁵: is a substituted aryl group of the formula (V), 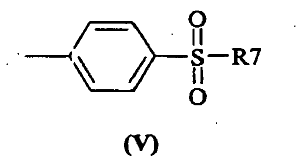 wherein R⁷ is a phenylamino, 2-pyridylamino, or 5-methylisoxasolyl-3-amino;
- R⁸: is hydrogen or acetyl; and
- X: is oxygen or sulfur;
or a pharmaceutically acceptable salt, solvate or ester thereof.

Particularly preferred compounds include:

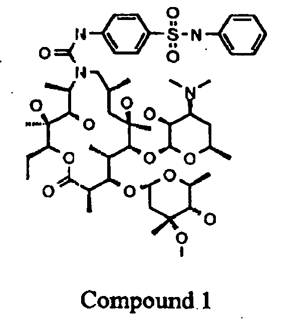

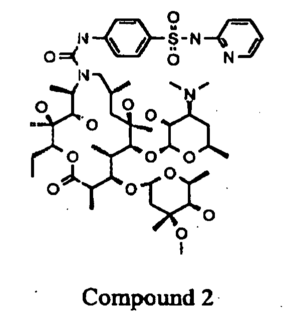

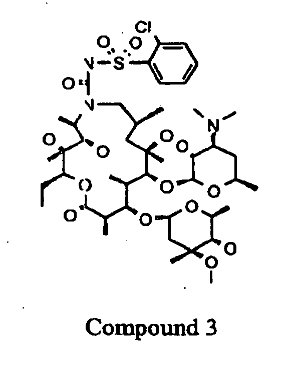

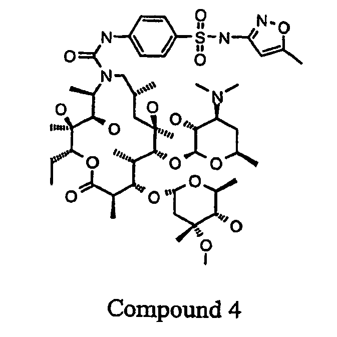

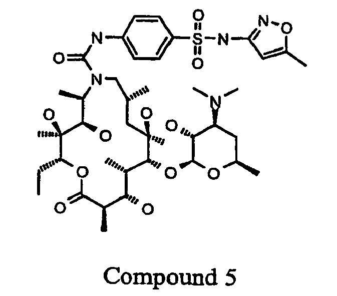

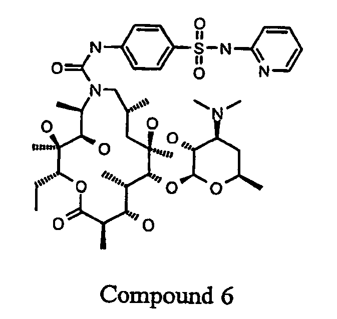

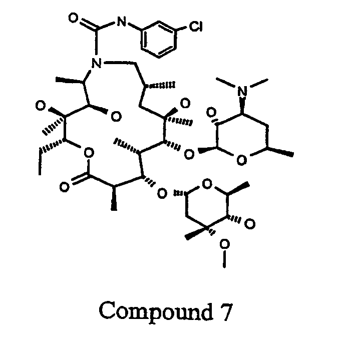

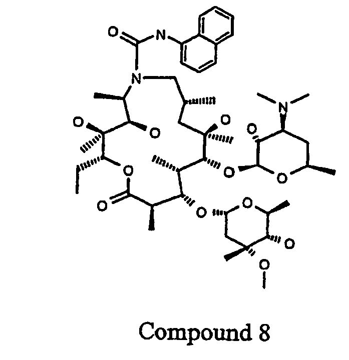

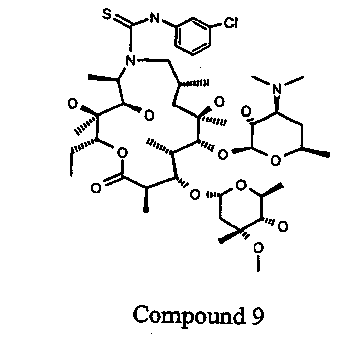

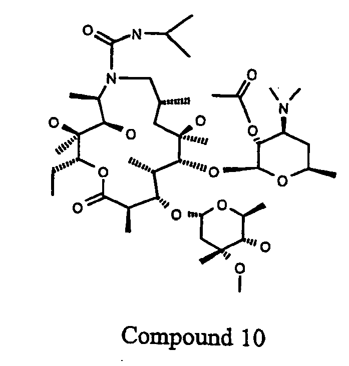

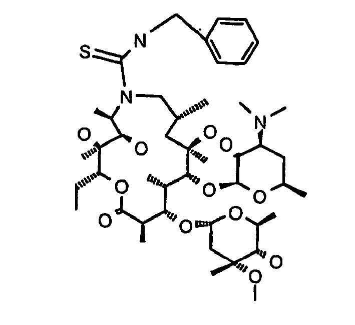

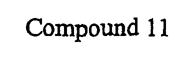

Azalide compounds described by Formula I may be prepared by methods described in US 5,629,296, EP1175429, EP 0657464, EP1414835 (WO 2002/068438 A2), WO 2004/043985, WO 2004/101591, each disclosure of which is incorporated by reference herein in its entirety. Particularly, the compounds may be prepared in accordance with the process described in EP1414835 on pages 4 - 5 and processes analogous to this synthesis. For example, instead of reacting the azalide intermediate with the phenylsulfonylisocyanate described in EP1414835 as general formula 3: 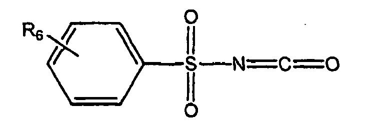 the azalide may be reacted instead with a compound described by:

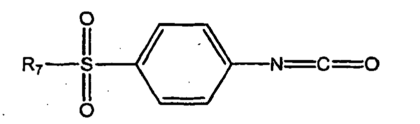

For the synthesis of the compounds described in Formula I. Other modifications of the reaction synthesis may be made based on standard organic chemical practices. For example, the compounds of Formula I are formed by reaction of the appropriate 9a-nitrogen unsubstituted azalide with the appropriate R₅-substituted isocyanate or thioisocyanate using the reaction conditions described in US 5,629,296, EP1175429, EP 0657464, EP1414835, WO 2004/043985 and/or WO 2004/101591.

It will be appreciated by those skilled in the art that it may be desirable to use protected derivatives of intermediates used in the preparation of the compounds of Formula I. Protection and deprotection of functional groups may be performed by methods known in the art. Hydroxyl or amino groups may be protected with any hydroxyl or amino protecting group (for example, as described in Green and Wuts. Protective Groups in Organic Synthesis. John Wiley and Sons, New York, 1999). The protecting groups may be removed by conventional techniques. For example, acyl groups (such as alkanoyl, alkoxycarbonyl and aryloyl groups) may be removed by solvolysis (e.g., by hydrolysis under acidic or basic conditions). Arylmethoxycarbonyl groups (e.g., benzyloxycarbonyl) may be cleaved by hydrogenolysis in the presence of a catalyst such as palladium-on-carbon.

The synthesis of the target compound is completed by removing any protecting groups, which are present in the penultimate intermediate using standard techniques, which are well-known to those skilled in the art. The deprotected final product is then purified, as necessary, using standard techniques such as silica gel chromatography, HPLC on silica gel and the like, or by recrystallization.

The term "salts" can include acid addition salts or addition salts of free bases. Examples of acids which may be employed to form pharmaceutically acceptable acid addition salts include but are not limited to salts derived from nontoxic inorganic acids such as nitric, phosphoric, sulfuric, or hydrobromic, hydroiodic, hydrofluoric, phosphorous, as well as salts derived from nontoxic organic acids such as aliphatic mono- and dicarboxylic acids, phenyl-substituted alkanoic acids, hydroxyl alkanoic acids, alkanedioic acids, aromatic acids, aliphatic and aromatic sulfonic acids, and acetic, maleic, succinic, or citric acids. Non-limiting examples of such salts include napadisylate, besylate, sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge S. M. et al. "Pharmaceutical Salts," J. of Pharma. Sci., 1977; 66:1).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine.

The base addition salts of said acidic compounds are prepared by contacting the free acid form with a sufficient amount of the desired base to produce the salt in the conventional manner. The free acid form may be regenerated by contacting the salt form with an acid and isolating the free acid.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). Preferably, as used herein, the term "pharmaceutically acceptable" means approved by a regulatory agency of the Federal or a state government or listed in the U.S. Pharmacopoeia or other generally recognized pharmacopeias for use in mammals, and more particularly in humans.

The term "pharmaceutically acceptable derivative" as used herein means any pharmaceutically acceptable salt, solvate or prodrug, e.g. ester, of a compound of the invention, which upon administration to the recipient is capable of providing (directly or indirectly) a compound of the invention, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. Preferred pharmaceutically acceptable derivatives are salts, solvates, esters, carbamates and phosphate esters. Particularly preferred pharmaceutically acceptable derivatives are salts, solvates and esters. Most preferred pharmaceutically acceptable derivatives are salts and esters.

The compounds of Formula I may be administered with one or more carriers. The term "carrier" applied to pharmaceutical compositions of the invention refers to a diluent, excipient, or vehicle with which an active compound is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by E.W. Martin, 18th Edition. Particularly preferred for the present invention are carriers suitable for immediate-release, i.e., release of most or all of the active ingredient over a short period of time, such as 60 minutes or less, and make rapid absorption of the drug possible.

The present invention also encompasses prodrugs of the Formula I compounds, i.e., compounds which release an active parent drug according to Formula I *in vivo* when administered to a mammalian subject. Prodrugs of a compound of Formula I are prepared by modifying functional groups present in the compound of Formula I in such a way that the modifications may be cleaved *in vivo* to release the parent compound. Prodrugs include compounds of Formula I wherein a hydroxy, amino, or carboxy group of a Formula I compound is bonded to any group that may be cleaved *in vivo* to regenerate the free hydroxyl, amino or carboxy group, respectively. Examples of prodrugs include, but are not limited to esters (e.g., acetate, formate, and benzoate derivatives) of compounds of Formula I or any other derivative which upon being brought to the physiological pH or through enzyme action is converted to the active parent drug.

The present invention also encompasses solvates of the compounds of Formula I or their salts. Preferred solvates are hydrates.

The compounds of Formula I have one or more chirality centers and, depending on the nature of individual substituents, they can also have geometrical isomers. Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has a chiral center, a pair of enantiomers is possible. An enantiomer can be characterized by the absolute configuration of its asymmetric center and is described by the R-- and S-sequencing rules of Cahn and Prelog, or by the manner in which the molecule rotates the plane of polarized light and designated as dextrorotatory or levorotatory (i.e., as (+) or (-)-isomer respectively). A chiral compound can exist as either an individual enantiomer or as a mixture of enantiomers. A mixture containing equal proportions of the enantiomers is called a "racemic mixture". The present invention encompasses all individual isomers of compounds of Formula I. The description or naming of a particular compound in the specification and claims is intended to include both individual enantiomers and mixtures, racemic or otherwise (i.e., enriched in one or more isomers) thereof. Methods for the determination of stereochemistry and the resolution of stereoisomers are well-known in the art.

The present invention also encompasses stereoisomers of the syn-anti type, and mixtures thereof encountered when an oxime or similar group is present. The group of highest Cahn Ingold Prelog priority attached to one of the terminal doubly bonded atoms of the oxime, is compared with hydroxyl group of the oxime. The stereoisomer is designated as *Z* (*zusammen* = together) or *Syn* if the oxime hydroxyl lies on the same side of a reference plane passing through the C=N double bond as the group of highest priority; the other stereoisomer is designated as *E* (*entgegen* = opposite) or *Anti*.

Depending on the type of formulation, in addition to a therapeutically effective quantity of one or more compounds of Formula I, they will contain solid or liquid excipients or diluents for pharmaceutical use and possibly other additives normally used in the preparation of pharmaceutical formulations, such as thickeners, aggregating agents, lubricants, disintegrating agents, flavorings and colorants.

The term "alkyl" as used herein as a group or a part of a group refers to a straight or branched hydrocarbon chain containing the specified number of carbon atoms. For example, C₁₋₆ alkyl means a straight or branched alkyl chain containing from 1 to 6 carbon atoms; examples of such group include methyl, ethyl, propyl, isopropyl, n-butyl, isobutyl, tert-butyl, pentyl, 3-methyl-butyl, hexyl and 2,3-dimethylbutyl and like. Similarly, the term C1-4 alkyl means a straight or branched alkyl chain containing from 1 to 4 carbon atoms.

The term "alkenyl" as used herein as a group or a part of a group refers to a straight or branched hydrocarbon chain containing the specified number of carbon atoms and containing at least one double bond. For example, the term "C₂₋₄ alkenyl" means a straight or branched alkenyl containing at least 2, and at most 4, carbon atoms and containing at least one double bond. Examples of "alkenyl" as used herein include, but are not limited to, ethenyl, 2-propenyl, 3-butenyl, 2-butenyl, 2-methyl-2-propenyl, 2-methylbut-2-ethenyl. It will be appreciated that in groups of the form -O-C₂₋₆ alkenyl, the double bond is preferably not adjacent to the oxygen.

The term "alkoxy", as used herein, refers to a straight or branched chain C₁₋₅ alkyl group, as previously defined, attached to the parent molecular moiety through an oxygen atom containing the specified number of carbon atoms. For example, C₁₋₄ alkoxy means a straight or branched alkoxy containing at least 1, and at most 4, carbon atoms. Examples of "alkoxy" as used herein include, but are not limited to, methoxy, ethoxy, propoxy, prop-2-oxy, butoxy, but-2-oxy, 2-methylprop-1-oxy and 2-methylprop-2-oxy.

The terms "haloalkyl" and "haloalkoxy" refers "alkyl" and "alkoxy" groups as defined above substituted with one are more halogen, where the halogen is a fluorine, chlorine, bromine or iodine atom.

"Treating" or "treatment" of malaria includes
(1) preventing or delaying the appearance of clinical symptoms of malaria developing in a mammal that has been in contact with the parasite.
(2) inhibiting the malaria, i.e., arresting, reducing or delaying the development of malaria or a relapse thereof or at least one clinical or subclinical symptom thereof, or
(3) relieving or attenuating one or more of the clinical or subclinical symptoms of malaria.

The benefit to a subject to be treated is either statistically significant or at least perceptible to the patient or to the physician.

"Prophylactic treatment" of malaria includes treating subjects who are at risk of developing malaria. This includes the treatment of subjects who have been exposed to malaria-bearing mosquitoes, the treatment of subjects who intend to travels to a country where malaria is endemic and the treatment of subjects who otherwise risk exposure to malaria-bearing mosquitoes.

An example of "relieving" a subclinical symptom is the observation in a treated individual of abatement in the number of immune cells that secrete pro inflammatory cytokines or lymphokines or a decrease in the mRNA encoding such lymphokines or cytokines.

"Maintenance therapy" is therapy during a phase of malaria following the acute phase, where the parasite achievement of remission (total or partial) of one or more symptoms of the disease until the next flare-up of the disease. The *Plasmodium vivax* and *P. ovale* parasites have dormant liver stages that can remain silent for years. Maintenance therapy for these strains is particularly important. The hallmarks of the acute phase include symptoms like chills, and fever.

"Responder" refers to a patient that has previously responded to a treatment for a non-infective inflammatory disease involving administration of a particular active agents (or combination of active agents) in particular amount or amounts.

"Subject" refers to an animal, which is preferably a mammal and more preferably human or a domestic animal. Most preferably, the subject is a human. As used herein, the term patient is used synonymously with subject.

A "therapeutically effective amount" means the amount of a compound that, when administered to a mammal for treating a state, disorder or condition, is sufficient to effect such treatment. The "therapeutically effective amount" will vary depending on the compound, the disease and its severity and the age, weight, physical condition and responsiveness of the mammal to be treated.

### Pharmaceutical Compositions

While it is possible that, for use in the methods of the invention, a compound of Formula I may be administered as the bulk substance, it is preferable to present the active ingredient in a pharmaceutical formulation, e.g., wherein the agent is in admixture with a pharmaceutically acceptable carrier selected with regard to the intended route of administration and standard pharmaceutical practice.

The phrase "pharmaceutically acceptable" refers to molecular entities and compositions that are generally regarded as safe. In particular, pharmaceutically acceptable carriers used in the pharmaceutical compositions of this invention are physiologically tolerable and do not typically produce an allergic or similar untoward reaction (for example, gastric upset, dizziness and the like) when administered to a patient. Preferably, as used herein, the term "pharmaceutically acceptable" means approved by a regulatory agency of the Federal or a state government or listed in the U.S. Pharmacopoeia or other generally recognized pharmacopoeia for use in animals, and more particularly in humans.

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

The term "carrier" refers to a diluent, excipient, and/or vehicle with which an active compound is administered. The pharmaceutical compositions of the invention may contain combinations of more than one carrier. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 18th Edition.

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in a conventional manner with the aid of one or more suitable carriers. Acceptable carriers for therapeutic use are well-known in the pharmaceutical art, and are described, for example, in Remington's Pharmaceutical Sciences, Mack Publishing Co. (A. R. Gennaro edit. 1985). The choice of pharmaceutical carrier can be selected with regard to the intended route of administration and standard pharmaceutical practice. The pharmaceutical compositions may comprise as, in addition to, the carrier any suitable binder(s), lubricant(s), suspending agent(s), coating agent(s), and/or solubilizing agent(s).

It will be appreciated that pharmaceutical compositions for use in accordance with the present invention may be in the form of oral, parenternal, transdermal, inhalation, sublingual, topical, implant, nasal, or enterally administered (or other mucosally administered) suspensions, capsules or tablets, which may be formulated in conventional manner using one or more pharmaceutically acceptable carriers or excipients.

There may be different composition/formulation requirements depending on the different delivery systems. It is to be understood that not all of the compounds need to be administered by the same route. Likewise, if the composition comprises more than one active component, then those components may be administered by different routes. By way of example, the pharmaceutical composition of the present invention may be formulated to be delivered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route. Alternatively, the formulation may be designed to be delivered by multiple routes.

The present invention further relates to pharmaceutical formulations containing a therapeutically effective quantity of a compound of Formula I or one of its salts mixed with a pharmaceutically acceptable vehicle. The pharmaceutical formulations of the present invention can be liquids that are suitable for oral and/or parenteral administration, for example, drops, syrups, solutions, injectable solutions that are ready for use or are prepared by the dilution of a freeze-dried product but are preferably solid or semisolid as tablets, capsules, granules, powders, pellets, pessaries, suppositories, creams, salves, gels, ointments; or solutions, suspensions, emulsions, or other forms suitable for administration by the transdermal route or by inhalation.

The compounds of the invention can be administered for immediate-, delayed-, modified-, sustained-, pulsed-or controlled-release applications.

The most preferred oral compositions are slow, delayed or positioned release (e.g., enteric especially colonic release) tablets or capsules. This release profile can be achieved without limitation by use of a coating resistant to conditions within the stomach but releasing the contents in the colon or other portion of the GI tract wherein a lesion or inflammation site has been identified. Or a delayed release can be achieved by a coating that is simply slow to disintegrate. Or the two (delayed and positioned release) profiles can be combined in a single formulation by choice of one or more appropriate coatings and other excipients. Such formulations constitute a further feature of the present invention.

Suitable compositions for delayed or positioned release and/or enteric coated oral formulations include tablet formulations film coated with materials that are water resistant, pH sensitive, digested or emulsified by intestinal juices or sloughed off at a slow but regular rate when moistened. Suitable coating materials include, but are not limited to, hydroxypropyl methylcellulose, ethyl cellulose, cellulose acetate phthalate, polyvinyl acetate phthalate, hydroxypropyl methylcellulose phthalate, polymers of metacrylic acid and its esters, and combinations thereof. Plasticizers such as, but not limited to polyethylene glycol, dibutylphthalate, triacetin and castor oil may be used. A pigment may also be used to color the film. Suppositories are be prepared by using carriers like cocoa butter, suppository bases such as Suppocire C, and Suppocire NA50 (supplied by Gattefossé Deutschland GmbH, D-Weil am Rhein, Germany) and other Suppocire type excipients obtained by interesterification of hydrogenated palm oil and palm kernel oil (C8-C18 triglycerides), esterification of glycerol and specific fatty acids, or polyglycosylated glycerides, and whitepsol (hydrogenated plant oils derivatives with additives). Enemas are formulated by using the appropriate active compound according to the present invention and solvents or excipients for suspensions. Suspensions are produced by using micronized compounds, and appropriate vehicle containing suspension stabilizing agents, thickeners and emulsifiers like carboxymethylcellulose and salts thereof, polyacrylic acid and salts thereof, carboxyvinyl polymers and salts thereof, alginic acid and salts thereof, propylene glycol alginate, chitosan, hydroxypropylcellulose, hydroxypropylmethylcellulose, hydroxyethylcellulose, ethylcellulose, methylcellulose, polyvinyl alcohol, polyvinyl pyrrolidone, N-vinylacetamide polymer, polyvinyl methacrylate, polyethylene glycol, pluronic, gelatin, methyl vinyl ether-maleic anhydride copolymer, soluble starch, pullulan and a copolymer of methyl acrylate and 2-ethylhexyl acrylate lecithin, lecithin derivatives, propylene glycol tatty acid esters, glycerin fatty acid esters, sorbitan fatty acid esters, polyoxyethylene sorbitan fatty acid esters, polyethylene glycol fatty acid esters, polyoxyethylene hydrated caster oil, polyoxyethylene alkyl ethers, and pluronic and appropriate buffer system in pH range of 6.5 to 8. The use of preservatives, masking agents is suitable. The average diameter of micronized particles can be between 1 and 20 micrometers, or can be less than 1 micrometer. Compounds can also be incorporated in the formulation by using their water-soluble salt forms.

Alternatively, materials may be incorporated into the matrix of the tablet e.g. hydroxypropyl methylcellulose, ethyl cellulose or polymers of acrylic and metacrylic acid esters. These latter materials may also be applied to tablets by compression coating.

Pharmaceutical compositions can be prepared by mixing a therapeutically effective amount of the active substance with a pharmaceutically acceptable carrier that can have different forms, depending on the way of administration. Pharmaceutical compositions can be prepared by using conventional pharmaceutical excipients and methods of preparation. The forms for oral administration can be capsules, powders or tablets where usual solid vehicles including lactose, starch, glucose, methylcellulose, magnesium stearate, di-calcium phosphate, mannitol may be added, as well as usual liquid oral excipients including, but not limited to, ethanol, glycerol, and water. All excipients may be mixed with disintegrating agents, solvents, granulating agents, moisturizers and binders. When a solid carrier is used for preparation of oral compositions (e.g., starch, sugar, kaolin, binders disintegrating agents) preparation can be in the form of powder, capsules containing granules or coated particles, tablets, hard gelatin capsules, or granules without limitation, and the amount of the solid carrier can vary (between 1 mg to 1g). Tablets and capsules are the preferred oral composition forms.

Pharmaceutical compositions containing compounds of the present invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols. Suitable oils include, for example, soybean oil, coconut oil, olive oil, safflower oil, cottonseed oil, and the like. For parenteral administration, the carrier can also be an oily ester such as ethyl oleate, isopropyl myristate, and the like. Compositions of the present invention may also be in the form of microparticles, microcapsules, liposomal encapsulates, and the like, as well as combinations of any two or more thereof.

Examples of pharmaceutically acceptable disintegrants for oral compositions useful in the present invention include, but are not limited to, starch, pre-gelatinized starch, sodium starch glycolate, sodium carboxymethylcellulose, croscarmellose sodium, microcrystalline cellulose, alginates, resins, surfactants, effervescent compositions, aqueous aluminum silicates and crosslinked polyvinylpyrrolidone.

Examples of pharmaceutically acceptable binders for oral compositions useful herein include, but are not limited to, acacia; cellulose derivatives, such as methylcellulose, carboxymethylcellulose, hydroxypropylmethylcellulose, hydroxypropylcellulose or hydroxyethylcellulose; gelatin, glucose, dextrose, xylitol, polymethacrylates, polyvinylpyrrolidone, sorbitol, starch, pre-gelatinized starch, tragacanth, xanthane resin, alginates, magnesium-aluminum silicate, polyethylene glycol or bentonite.

Examples of pharmaceutically acceptable fillers for oral compositions include, but are not limited to, lactose, anhydrolactose, lactose monohydrate, sucrose, dextrose, mannitol, sorbitol, starch, cellulose (particularly microcrystalline cellulose), dihydro- or anhydro-calcium phosphate, calcium carbonate and calcium sulfate.

Examples of pharmaceutically acceptable lubricants useful in the compositions of the invention include, but are not limited to, magnesium stearate, talc, polyethylene glycol, polymers of ethylene oxide, sodium lauryl sulfate, magnesium lauryl sulfate, sodium oleate, sodium stearyl fumarate, and colloidal silicon dioxide.

Examples of suitable pharmaceutically acceptable odorants for the oral compositions include, but are not limited to, synthetic aromas and natural aromatic oils such as extracts of oils, flowers, fruits (*e*.*g*., banana, apple, sour cherry, peach) and combinations thereof, and similar aromas. Their use depends on many factors, the most important being the organoleptic acceptability for the population that will be taking the pharmaceutical compositions.

Examples of suitable pharmaceutically acceptable dyes for the oral compositions include, but are not limited to, synthetic and natural dyes such as titanium dioxide, beta-carotene and extracts of grapefruit peel.

Suitable examples of pharmaceutically acceptable sweeteners for the oral compositions include, but are not limited to, aspartame, saccharin, saccharin sodium, sodium cyclamate, xylitol, mannitol, sorbitol, lactose and sucrose.

Suitable examples of pharmaceutically acceptable buffers include, but are not limited to, citric acid, sodium citrate, sodium bicarbonate, dibasic sodium phosphate, magnesium oxide, calcium carbonate and magnesium hydroxide.

Suitable examples of pharmaceutically acceptable surfactants include, but are not limited to, sodium lauryl sulfate and polysorbates.

Suitable examples of pharmaceutically acceptable preservatives include, but are not limited to, various antibacterial and antifungal agents such as solvents, for example ethanol, propylene glycol, benzyl alcohol, chlorobutanol, quaternary ammonium salts, and parabens (such as methyl paraben, ethyl paraben, propyl paraben, etc.).

Suitable examples of pharmaceutically acceptable stabilizers and antioxidants include, but are not limited to, ethylenediaminetetriacetic acid (EDTA), thiourea, tocopherol and butyl hydroxyanisole.

The compounds of the invention may also, for example, be formulated as suppositories *e*.*g*., containing conventional suppository bases for use in human or veterinary medicine or as pessaries *e*.*g*., containing conventional pessary bases.

The compounds according to the invention may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops (*e*.*g*., eye ear or nose drops) or pour-ons.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax, sorbitan monostearate, a polyethylene glycol, liquid paraffin, polysorbate 60, cetyl esters wax, cetearyl alcohol, 2-octyldodecanol, benzyl alcohol, and water. Such compositions may also contain other pharmaceutically acceptable excipients, such as polymers, oils, liquid carriers, surfactants, buffers, preservatives, stabilizers, antioxidants, moisturizers, emollients, colorants, and odorants.

Examples of pharmaceutically acceptable polymers suitable for such topical compositions include, but are not limited to, acrylic polymers; cellulose derivatives, such as carboxymethylcellulose sodium, methylcellulose or hydroxypropylcellulose; natural polymers, such as alginates, tragacanth, pectin, xanthan and cytosan.

As indicated, the compound of the present invention can be administered intranasally or by inhalation and is conveniently delivered in the form of a dry powder inhaler or an aerosol spray presentation from a pressurized container, pump, spray or nebulizer with the use of a suitable propellant, *e*.*g*., dichlorodifluoromethane, trichlorofluoromethane, dichlorotetrafluoroethane, a hydrofluoroalkane such as 1,1,1,2-tetrafluoroethane (HFA 134AT"") or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebulizer may contain a solution or suspension of the active compound, *e*.*g*., using a mixture of ethanol and the propellant as the solvent, which may additionally contain a lubricant, *e*.*g*., sorbitan trioleate.

Capsules and cartridges (made, for example, from gelatin) for use in an inhaler or insufflator may be formulated to contain a powder mix of the compound and a suitable powder base such as lactose or starch.

For topical administration by inhalation the compounds according to the invention may be delivered for use in human or veterinary medicine via a nebulizer.

The pharmaceutical compositions of the invention may contain from 0.01 to 99% weight per volume of the active material. For topical administration, for example, the composition will generally contain from 0.01-10%, more preferably 0.01-1% of the active material.

A therapeutically effective amount of the compound of the present invention can be determined by methods known in the art. The therapeutically effective quantities will depend on the age and on the general physiological condition of the patient, the route of administration and the pharmaceutical formulation used. It will also be determine by the strain of malaria parasite that has infected the subject. The therapeutic doses will generally be between about 10 and 2000 mg/day and preferably between about 30 and 1500 mg/day. Other ranges may be used, including, for example, 50-500 mg/day, 50-300 mg/day, 100-200 mg/day. The amount of the compound required for prophylactic treatment, referred to as a prophylactically-effective dosage, is generally the same as described for therapeutic treatment.

Administration may be once a day, twice a day, or more often, and may be decreased during a maintenance phase of the disease or disorder, e.g. once every second or third day instead of every day or twice a day. The dose and the administration frequency will depend on the clinical signs, which confirm maintenance of the remission phase, with the reduction or absence of at least one or more preferably more than one clinical signs of the acute phase know to the person skilled in the art.

### Examples

The features and advantages of the invention are more fully shown by the following non-limiting examples.

The therapeutic effect of compounds of the present invention was determined in experiments provided in the examples.

### Example 1

### In vitro Screening Protocols

The *in vitro* screens for intrinsic antimalarial activity were based on modifications of the procedures described by Desjardins RE, Canfield CJ, Haynes JD, Chulay JD. (Quantitative assessment of antimalarial activity in vitro by a semiautomated microdilution technique. Antimicrob Agents Chemother. 1979 Dec;16(6):710-8.), Chulay JD, Haynes JD, Diggs CL. (*Plasmodium falciparum*: Assessment of in vitro growth by [₃H]hypoxanthine incorporation. Exp. Parasitol. 1983 55:138-146.), and Milhous WK, Weatherly NF, Bowdre JH, Desjardins RE. (In vitro activities of and mechanisms of resistance to antifol antimalarial drugs. Antimicrob Agents Chemother. 1985 Apr;27(4):525-30.). The system was limited to the assessment of the intrinsic activity against the erythrocytic asexual life cycle (blood schizontocides). Two *Plasmodium falciparum* clones from CDC/Indochina III (W-2) and CDC/Sierra Leone I (D-6) (Oduola AM, Weatherly NF, Bowdre JH, Desjardins RE. Plasmodium falciparum: cloning by single-erythrocyte micromanipulation and heterogeneity in vitro. Exp Parasitol. 1988 66(1):86-95.) were used for all assays. TM91C235, a multiple drug resistant isolate from Thailand, was used for the prescreening assay. W-2 is resistant to chloroquine, quinine, and pyrimethamine and susceptible to mefloquine. D-6 tends to be more resistant to mefloquine and susceptible to chloroquine, quinine, and pyrimethamine. All documents cited in this paragraph are incorporated by reference in their entirety.

All parasites were maintained in continuous long term cultures in RPMI-1640 medium supplemented with 6% washed human A positive (A+)(erythrocytes, 25 mM Hepes, 32 nM NaHCO₃, and 10% heat inactivated A+ human plasma or ALBUMAX® (lipid-rich bovine serum albumin; Invitrogen, Carlsbad, CA). All cultures and assays were conducted at 37°C under an atmosphere of 5% CO₂ and 5% O₂, with a balance of N₂.

### Example 2

### PreScreening Assay

The prescreening assay uses TM91C235 diluted at a 0.4% parasitemia in a 1% hematocrit in folic acid free and p-aminobenzoic acid free media RPMI-1640 and ALBUMAX®. One mg of the compound is typically dissolved in 100 µl of dimethyl sulfoxide (DMSO). The compound is further diluted in culture medium (FF) with ALBUMAX® for the first initial starting concentration. The rest of the stock drug solution was kept at -70°C. The isolate was preexposed, in duplicate, at three concentrations (25,000 ng/ml, 2,500 ng/ml, and 25 ng/ml) of the test compound for 48 hr in a 96-well microtiter plate (MTP) using the BIOMEK® 2000 automated laboratory workstation. Each MTP contains chloroquine as control to assess the relative activity of the compound and to monitor the response of TM91 C235.

After the preincubation, [³H]-hypoxanthine was added to each well of the MTP. (The assay relies on the incorporation of radiolabeled hypoxanthine by the parasites, which indicates reproduction, and inhibition of isotope incorporation was attributed to activity of known or candidate antimalarial drugs). After 72 hr of total incubation time, the MTP were frozen to lyse the erythrocytes and parasites. The parasite DNA was recovered by harvesting the lysate onto glass-fiber filter plates using a Packard FilterMate™ Cell Harvester. The radioactivity was counted on a Packard TopCount™ microplate scintillation counter. The results were recorded as counts per minutes (CPM) per well at each drug concentration divided by the arithmetic mean of the CPM from the three untreated infection parasite control wells.

### Example 3

### Serial Dilution Assay

If a compound did not affect parasite growth at 25,000 ng/ml, it was classified as inactive. If a compound suppressed greater than two standard deviations from the arithmetic mean of the untreated infection controls at 25,000 ng/ml, but less than 50% at 2,500 ng/ml, the compound was designated as partially active. However, if a compound suppressed greater than 50% of the incorporation of [³H]- hypoxanthine relative to untreated infection control parasites at 2,500 ng/ml, the compound was classified as fully active and was further evaluated by two-fold serial dilutions to determine the IC₅₀ value (50% inhibitory concentration).

The serial dilution assay was conducted using the same assay conditions and stock solution of the compound used for the preliminary screen, described above. Both the D-6 and the W-2 clones were used. The compounds were diluted two-fold over 11 different concentration ranges with a starting concentration that was based on the preliminary screen. For each drug, the concentration response profile was determined and 50% inhibitory concentrations (IC₅₀) were determined by using a nonlinear, logistic dose response analysis program. If the results from this assay did not agree with the concentration ranges of the preliminary screen, the assay was repeated. For each assay, the IC₅₀ for each clone was determined against the known antimalarials chloroquine and mefloquine. These control values established the compound's relative parasite susceptibility profile compared to known antimalarials.

### Example 4

IC₅₀s can be similarly determined for drug-resistant isolates/clones from a wide variety of geographic locations by using different isolates/clones in the assays described herein. The assays described in Examples 1 - 3 can be repeated using both samples according to Formula I and isolates/clones from different malaria strains to determine the antimalarial activity of the compounds. For Example, the above assays can be used to determine the IC₅₀ values for malarial strains TM91C235 (reported to be resistant to mefloquine, chloroquine, and quinine), D6 (reported to be resistant to mefloquine), and W2 (reported to be resistant to chloroquine, quinine, and pyrimethamine).

### Example 5

### Assay Results

As described above, parasites were exposed for 72 hours. Data from the *in vitro* potency tests of azithromycin against the three parasite strains have been averaged, and these numbers were used for identification of "active" macrolide compounds. These average IC₅₀s against the TM91C235, D6, and W2 strains were 1621.2 ng/mL, 796.9 ng/mL, and 1759.2 ng/mL, respectively.

**Table 1. Structures of the selected tested compounds.**

| Compound number | Structure |
|---|---|
| 1 | 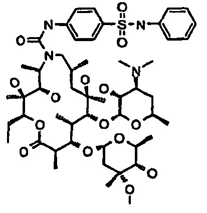 |
| 2 | 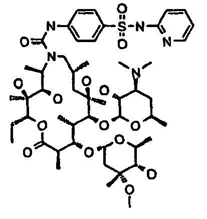 |
| 3 | 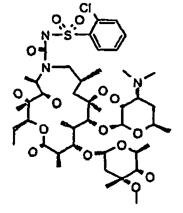 |
| 4 | 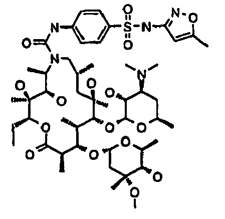 |
| 5 | 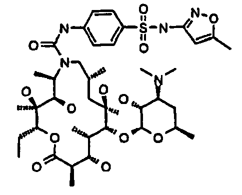 |
| 6 | 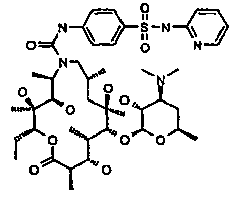 |
| 7 | 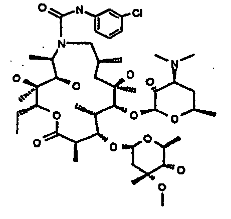 |
| 8 | 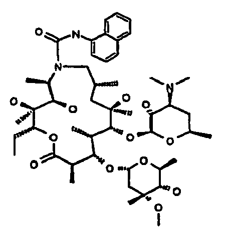 |
| 9 | 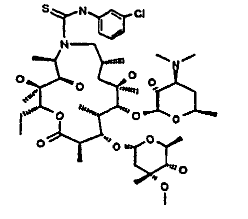 |
| 10 | 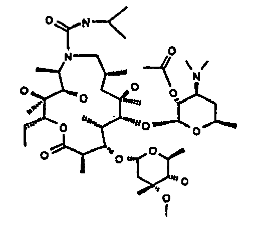 |
| 11 | 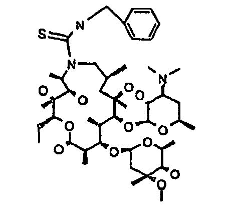 |

**Table 2: IC₅₀ Values of macrolide compounds, in comparison with azithromycin, tested against the three parasite strains (TM91C235, D6, and W2) with different patterns of resistance.**

| Compound | IC₅₀ (ng/mL) | | |
|---|---|---|---|
| | TM91C235 | D6 | W2 |
| azithromycin | 1621.2 | 796.9 | 1759.2 |
| Compound 1 | 299.2 | 427.9 | 447.7 |
| Compound 2 | 1027.4 | 250.7 | NA |
| Compound 3 | NA | 1111.2 | 1433.0 |
| Compound 4 | NA | 119.7 | 1588.2 |
| Compound 5 | NA | 52.3 | NA |
| Compound 6 | NA | 90.8 | NA |
| Compound 7 | ND | 582.5 | 1219.4 |
| Compound 8 | 712.1 | 750.9 | 1904.4 |
| Compound 9 | ND | 762.3 | 1501.5 |
| Compound 10 | 353.4 | NA | NA |
| Compound 11 | 304.4 | 253.0 | 385.5 |

| | | | |
|---|---|---|---|
| NA: less active than azithromycin; ND: not determined | | | |

## Claims

1. Use of a compound of formula (I) 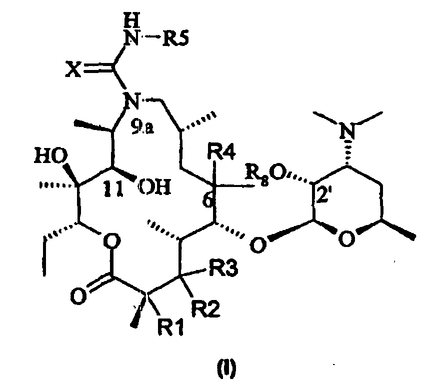 wherein:
R¹ is hydrogen or together with R² is a double bond;
R² is a cladinose sugar of formula (II), hydrogen, hydroxyl or group of the formula (III) wherein Y is a monocyclic aromatic ring unsubstituted or substituted with one or more groups which are selected from halogen, OH, OMe, NO₂, and NH₂; or 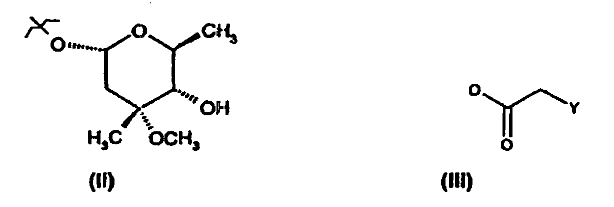
R² together with R³ is a ketone, or together with R¹ is a double bond;
R³ is hydrogen or together with R² is a ketone, or together with R⁴ is an ether;
R⁴ is hydroxyl or OMe, or together with R³ is an ether;
R⁵ is C₁₋₄alkyl, C₂₋₄alkenyl, -(CH₂)ₘ-Ar, wherein Ar is a monocyclic or bicyclic aromatic ring having up to 10 carbon atoms, containing 0-3 heteroatoms selected from N and O, unsubstituted or substituted by one or more of halogen, C₁₋₆haloalkyl, C₁₋₄haloalkoxy, C₁₋₆alkyl, or C₁₋₆alkoxy, and m is 0-3; or
R⁵ is a substituted aryl group of the formula (IV), 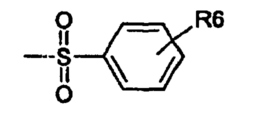 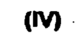 wherein R⁸ is H, C₁₋₄ alkyl or halogen,
or,
R⁶ is a substituted aryl group of the formula (V), 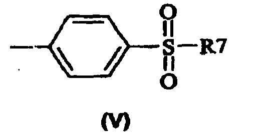 wherein R⁷ is chloro, amino, phenylamino, 2-pyridylamino, 3,4-dimethylisoxazolyl-5-amino, or 5-methylisoxazolyl-3-amino;
R⁸ is hydrogen or hydroxyl protecting group; and
X is oxygen or sulfur,
or a pharmaceutically acceptable salt, solvate or ester thereof, in the manufacture of a medicament for the therapeutic and/or prophylactic treatment of malaria.

2. Use of claim 1, wherein the subject has been infected with *Plasmodium falciparum*.

3. Use of claim 1, wherein the subject has been infected with *P. vivax*.

4. Use of claim 1, wherein the subject has been infected with *P. ovale*.

5. Use of claim 1, wherein the subject has been infected with *P. malariae*.

6. Use of claim 1, wherein
R¹ is hydrogen;
R² is hydroxyl, or a cladinose sugar of formula (II); 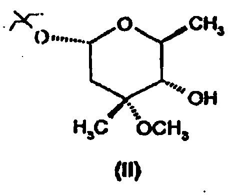
R³ is hydrogen;
R⁴ is hydroxyl;
R⁵ is isopropyl, benzyl, 3-chlorophenyl or 1-naphthyl; or
R⁵ Is a substituted aryl group of the formula (IV), 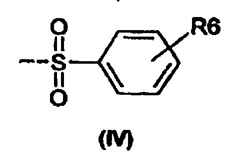 wherein R⁶ is 2-chloro,
or,
R⁶ is a substituted aryl group of the formula (V), 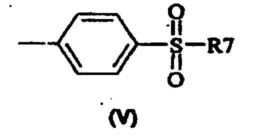 wherein R⁷ is a phanylamino, 2-pyridylamino, or 5-methylisoxazolyl-3-amino;
R⁵ Is hydrogen or acetyl; and
X is oxygen or sulfur;
or a pharmaceutically acceptable salt, solvate or ester thereof.

7. The use of claim 1, wherein the 9a-carbamoyl or thiocarbamoyl azalide is selected from: 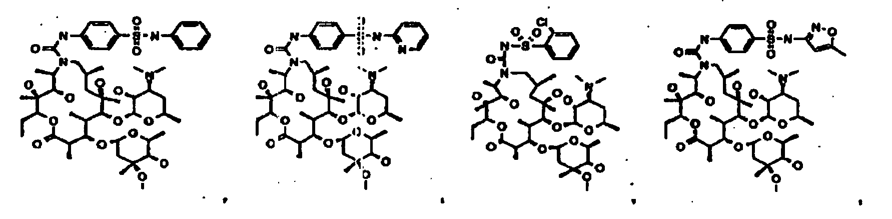 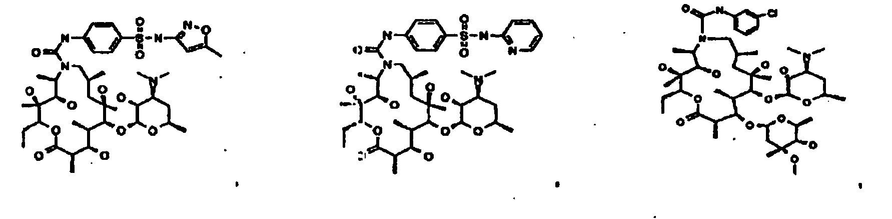 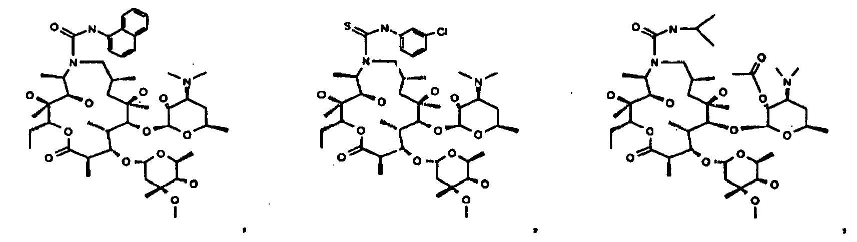 and 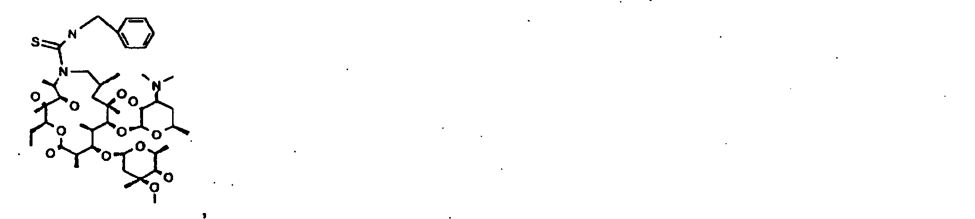 or a pharmaceutically acceptable salt, solvate or ester thereof.

8. Use of claim 1, wherein the medicament is administered after the subject has been exposed to the malaria parasite.

9. Use of claim 8, wherein the malaria parasite is a drug-resistant malarial strain.

10. Use of claim 9, wherein the drug-resistant malarial strain is resistant to one or more of chloroquine, mefloquine, halofantrine, artemisinin, atovaquone/proguanil, doxycycline or primaquine.

11. Use of claim 1, wherein the medicament is administered before the subject travels to a country where malaria is endemic.

## Patentansprüche

1. Verwendung einer Verbindung der Formel **(I)** 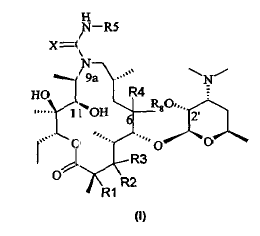 wobei:
R¹ Wasserstoff oder zusammen mit R² eine Doppelbindung ist;
R² ein Cladinose-Zucker der Formel **(II),** Wasserstoff, Hydroxyl oder ein Rest der Formel **(III)** ist, wobei Y ein monocyclischer aromatischer Ring ist, unsubstituiert oder mit einem oder mehreren Resten substituiert, welche ausgewählt sind Halogen, OH, OMe, NO₂ und NH₂; oder 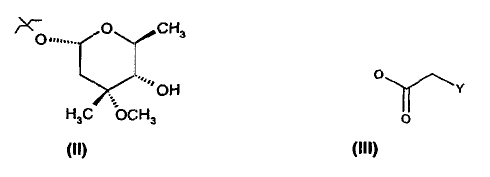
R² zusammen mit R³ ein Keton oder zusammen mit R¹ eine Doppelbindung ist;
R³ Wasserstoff oder zusammen mit R² ein Keton oder zusammen mit R⁴ ein Ether ist;
R⁴ Hydroxyl oder OMe oder zusammen mit R³ ein Ether ist;
R⁵ C₁₋₄-Alkyl, C₂₋₄-Alkenyl, -(CH₂)ₘ-Ar ist, wobei Ar ein monocyclischer oder bicyclischer aromatischer Ring mit bis zu 10 Kohlenstoffatomen ist, welcher 0 bis 3 Heteroatome, ausgewählt aus N und O, enthält, unsubstituiert oder substituiert mit einem oder mehreren aus Halogen, C₁₋₆-Halogenalkyl, C₁₋₆-Halogenalkoxy, C₁₋₆-Alkyl oder C₁₋₆-Alkoxy, und m 0 bis 3 ist; oder
R⁵ ein substituierter Arylrest der Formel **(IV)** 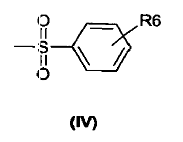 ist,
wobei R⁶ H, C₁₋₄-Alkyl oder Halogen ist;
oder
R⁵ ein substituierter Arylrest der Formel **(V)** 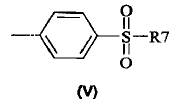 ist, wobei R⁷ Chlor, Amino, Phenylamino, 2-Pyridylamino, 3,4-Dimethylisoxazolyl-5-amino oder 5-Methylisoxazolyl-3-amino ist;
R⁸ Wasserstoff oder eine Hydroxylschutzgruppe ist; und
X Sauerstoff oder Schwefel ist;
oder eines pharmazeutisch verträglichen Salzes, Solvats oder Esters davon, zur Herstellung eines Medikaments zur therapeutischen und/oder prophylaktischen Behandlung von Malaria.

2. Verwendung gemäß Anspruch 1, wobei das Subjekt mit *Plasmodium falciparum* infiziert worden ist.

3. Verwendung gemäß Anspruch 1, wobei das Subjekt mit *P. vivax* infiziert worden ist.

4. Verwendung gemäß Anspruch 1, wobei das Subject mit *P. ovale* infiziert worden ist.

5. Verwendung gemäß Anspruch 1, wobei das Subjekt mit *P. malariae* infiziert worden ist.

6. Verwendung gemäß Anspruch 1, wobei
R¹ Wasserstoff ist;
R² Hydroxyl oder ein Cladinose-Zucker der Formel **(II):** 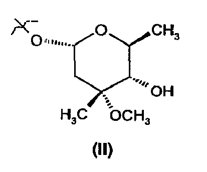 ist;
R³ Wasserstoff ist;
R⁴ Hydroxyl ist;
R⁵ Isopropyl, Benzyl, 3-Chlorphenyl oder 1-Naphthyl ist; oder
R⁵ ein substituierter Arylrest der Formel **(IV)** 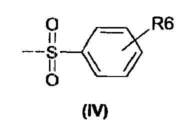 ist,
wobei R⁶ 2-Chlor ist,
oder
R⁵ ein substituierter Arylrest der Formel **(V)** 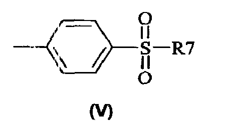 ist,
wobei R⁷ Phenylamino, 2-Pyridylamino oder 5-Methylisoxazolyl-3-amino ist;
R⁸ Wasserstoff oder Acetyl ist; und
X Sauerstoff oder Schwefel ist;
oder eines pharmazeutisch verträglichen Salzes, Solvats oder Esters davon.

7. Verwendung gemäß Anspruch 1, wobei das 9a-Carbamoyl oder Thiocarbamoylazalid ausgewählt ist aus: 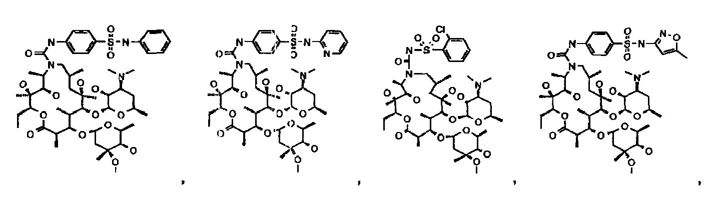 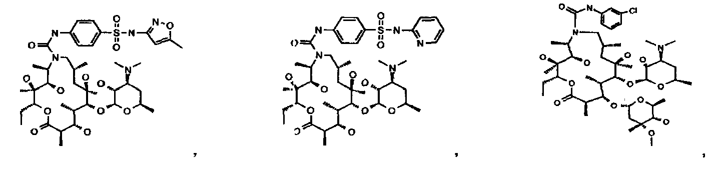 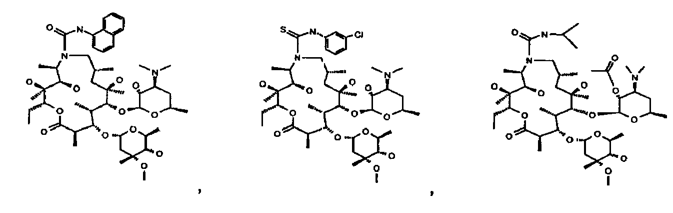 und 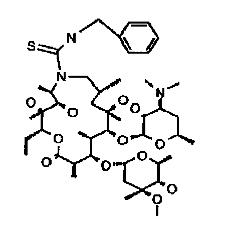 oder einem pharmazeutisch verträglichen Salz, Solvat oder Ester davon.

8. Verwendung gemäß Anspruch 1, wobei das Medikament verabreicht wird, nachdem das Subjekt Malariaparasiten ausgesetzt worden ist.

9. Verwendung gemäß Anspruch 8, wobei der Malariaparasit ein Medikamenten-resistenter Malariastamm ist.

10. Verwendung gemäß Anspruch 9, wobei der Medikamenten-resistente Malariastamm gegen einen oder mehrere aus Chloroquin, Mefloquin, Halofantrin, Artemisinin, Atovaquon/Proguanil, Doxycyclin oder Primaquin resistent ist.

11. Verwendung gemäß Anspruch 1, wobei das Medikament verabreicht wird, bevor das Subjekt in ein Land reist, in dem Malaria endemisch ist.

## Revendications

1. Utilisation d'un composé de formule (I) 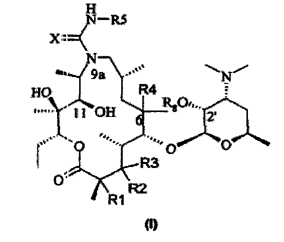 dans laquelle :
R¹ représente un atome hydrogène ou, conjointement avec R², représente une double liaison ;
R² représente un sucre cladinose de formule (II), un atome d'hydrogène, un groupe hydroxyle ou un groupe de formule (III) dans laquelle Y représente un noyau aromatique monocyclique, non substitué ou substitué avec un ou plusieurs groupes qui sont choisis entre des groupes halogéno, OH, OMe, NO₂ et NH₂ ; ou 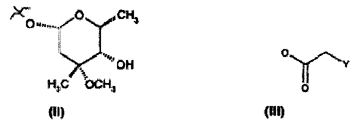
R² conjointement avec R³, représente un groupe cétone, ou bien, conjointement avec R¹, représente une double liaison ;
R³ représente un atome d'hydrogène ou, conjointement avec R², représente un groupe cétone, ou bien, conjointement avec R⁴, représente un groupe éther ;
R⁴ représente un groupe hydroxyle ou OMe ou, conjointement avec R³, représente un groupe éther ;
R⁵ représente un groupe alkyle en C₁ à C₄, alcényle en C₂ à C₄, -(CH₂)ₘ-Ar, dans lequel Ar représente un noyau aromatique monocyclique ou bicyclique ayant jusqu'à 10 atomes de carbone, contenant 0 à 3 hétéroatomes choisis entre N et O, non substitué ou substitué avec un ou plusieurs groupes halogéno, halogénalkyle en C₁ à C₆, halogénalkoxy en C₁ à C₆, alkyle en C₁ à C₆ ou alkoxy en C₁ à C₆, et m a une valeur de 0 à 3 ; ou
R⁵ représente un groupe aryle substitué de formule (IV), 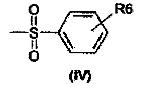 dans laquelle R⁶ représente H, un groupe alkyle en C₁ à C₄ ou halogéno,
ou
R⁵ représente un groupe aryle substitué de formule (V) 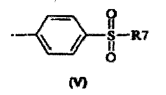 dans laquelle R⁷ représente un groupe chloro, amino, phénylamino, 2-pyridylamino, 3,4-diméthylisoxazolyl-5-amino, ou 5-méthylisoxazolyl-3-amino ;
R⁸ représente un atome d'hydrogène ou un groupe protecteur de la fonction hydroxyle ; et
X représente un atome d'oxygène ou de soufre ;
ou d'un de ses sels, produits de solvatation ou esters pharmaceutiquement acceptables, dans la production d'un médicament destiné au traitement thérapeutique et/ou prophylactique du paludisme.

2. Utilisation suivant la revendication 1, dans laquelle le sujet a été infecté par *Plasmodium falciparum.*

3. Utilisation suivant la revendication 1, dans laquelle le sujet a été infecté par *P. vivax.*

4. Utilisation suivant la revendication 1, dans laquelle le sujet a été infecté par *P. ovale*.

5. Utilisation suivant la revendication 1, dans laquelle le sujet a été infecté par *P. malariae.*

6. Utilisation suivant la revendication 1, dans laquelle
R¹ représente un atome d'hydrogène ;
R² représente un groupe hydroxyle, ou un sucre cladinose de formule (II) ; 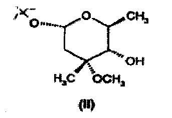
R³ représente un atome d'hydrogène ;
R⁴ représente un groupe hydroxyle ;
R⁵ représente un groupe isopropyle, benzyle, 3-chlorophényle ou 1-naphtyle ; ou
R⁵ représente un groupe aryle substitué, de formule (IV), 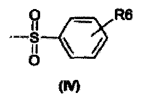 dans laquelle R⁶ représente un groupe 2-chloro,
ou
R⁵ représente un groupe aryle substitué, de formule (V), 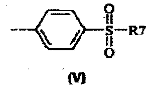 dans laquelle R⁷ représente un groupe phénylamino, 2-pyridylamino ou 5-méthylisoxazolyl-3-amino ;
R⁸ représente un atome d'hydrogène ou un groupe acétyle ; et
X représente un atome d'oxygène ou de soufre ;
ou d'un sel, produit de solvatation ou ester pharmaceutiquement acceptables du composé.

7. Utilisation suivant la revendication 1, dans laquelle le 9a-carbamoyl- ou -thiocarbamoyl-azalide est choisi entre : 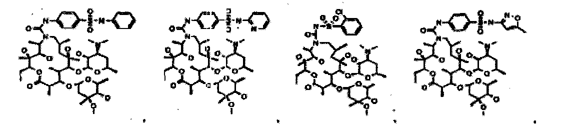 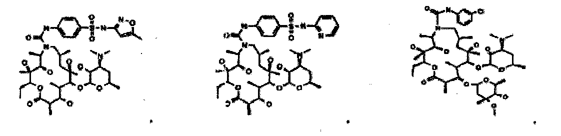 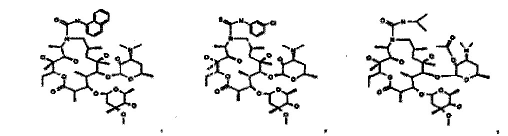 et 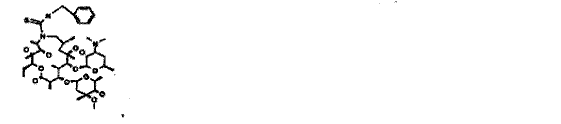 ou un de leurs sels, produits de solvatation ou esters pharmaceutiquement acceptables.

8. Utilisation suivant la revendication 1, dans laquelle le médicament est administré après exposition du sujet au parasite du paludisme.

9. Utilisation suivant la revendication 8, dans laquelle le parasite du paludisme est une souche de parasite du paludisme résistante aux médicaments.

10. Utilisation suivant la revendication 9, dans laquelle la souche du parasite du paludisme résistante aux médicaments est résistante à à un ou plusieurs des médicaments consistant en chloroquine, méfloquine, halofantrine, artémisinine, atovaquone/proguanil, doxycycline et primaquine.

11. Utilisation suivant la revendication 1, dans laquelle le médicament est administré avant que le sujet ne voyage dans un pays où le paludisme est endémique.
